# EUROPEAN PATENT APPLICATION

(11) **EP 2 090 649 A1**
(43) Date of publication of application: **19.08.2009**
(21) Application number: 08101576.0
(22) Date of filing: 13.02.2008
(51) Int. Cl.: C12N 5/06, C12N 5/08

(54) **Method for reprogramming differentiated cells**

(71) Applicant: Fondazione Telethon, 00154 Roma (IT)
(72) Inventor: Luiss Vinas, Frederic, 80138, Napoli (IT); Cosma, Maria Pia, 80134, Napoli (IT)
(74) Representative: Capasso, Olga

(57) **Abstract**

The present invention discloses a method for reprogramming a differentiated cell to an undifferentiated stem cell comprising fusing a pluripotent cell with a differentiated cell to form a fused cell, wherein the pluripotent cell is pre-treated or the fused cell is treated with a suitable amount of a Wnt/β-catenin pathway activator.

## Description

### Field of the invention

The present invention relates to a method for reprogramming a differentiated cell to an undifferentiated stem cell, the reprogrammed cell obtained thereby, the use thereof as well as the use of a Wnt/β-catenin activator or of a GSK-3 inhibitor for reprogramming a differentiated cell to an undifferentiated stem cell.

### Background of the invention

After their fusion, highly differentiated cells like sperm cells and oocytes generate pluripotent and undifferentiated zygotes. This is due to their reprogramming, a biological mechanism that can allow de-differentiation of differentiated cells. A somatic nucleus can be subjected to epigenetic modifications and reprogramming by fusing somatic mouse or human cells with embryonic stem (ES) cells ¹⁻³. Nanog is a factor that maintains ES cells undifferentiated and augments pluripotency of hybrids after fusion of ES and neural stem (NS) cells ⁴. Recent findings have shown that over-expression of four specific ES-cell genes in mouse and human fibroblasts can induce fibroblasts to become pluripotent cells ⁵⁻⁷. All factors analyzed to date that can induce reprogramming of somatic cells are ES-cell factors.

Patent application WO 2007/115216 provides methods for therapeutically reprogramming human adult stem cells into pluripotent cells. Such methods consist in isolating a human adult stem cell and placing it in contact with a medium comprising stimulatory factors which induce the development of the stem cell into a therapeutically reprogrammed cell. The medium comprises PM-10^{™}. Such method has been also proposed in the patent application WO 2005/123123 wherein the stimulatory factor is a chemical selected from the group consisting of 5-aza-2'-deoxycytidine, histone de-acetylase inhibitor, n-butyric acid and trichostatin.

Patent application WO 2007/054720 discloses methods for reprogramming and genetically modifying cells. In this application a pluripotent genome is obtained from a differentiated genome by fusing a pluripotent cell with a differentiated cell in the presence of Nanog or a MEK inhibitor.

Patent application WO 2007/019398 provides improved methods for reprogramming an animal differentiated somatic cell to an undifferentiated stem cell. Such methods comprise (a) injecting one or more animal differentiated somatic cells into an oocyte, (i) remodelling the nuclear envelope of the nucleus of the somatic cell, and (ii) reprogramming the chromatin of the somatic cell and (b) transferring or fusing the resulting remodelled nucleus obtained in (a) into the enucleated cytoplasm of an undifferentiated embryonic cell to form an undifferentiated stem cell.

Patent application WO 2007/016245 relates to methods of reprogramming adult stem cells or stem cells obtained from umbilical or placental cord blood or from the placenta or umbilical cord tissue itself or the amniotic fluid or amnion. The reprogrammed stem cells are obtained by exposing adult pluripotent stem or progenitor cells or pluripotent stem or progenitor cells obtained from placental or umbilical cord tissue or placental or umbilical cord blood or amniotic fluid or amnion to an effective amount of embryonic stem cell medium or to a denucleation procedure to remove nuclear DNA from the stem cell followed by introduction of nuclear material from a cell of a patient to be treated. The embryonic stem cell medium is a minimum essential medium comprising at least one growth factor, glucose, nonessential amino acids, insulin and transferrin. The growth factor is fibroblast growth factor, transforming growth factor beta or mixtures thereof and said medium further comprises at least one additional component selected from the group consisting gamma amino butyric acid, pipecholic acid, lithium and mixtures thereof.

Patent application WO 2005/033297 discloses compositions, methods and kits for non-nuclear transfer reprogramming an adult differentiated cell obtained from an adult tissue into an ES-like cell. The method of producing a reprogrammed embryonic stem-cell like cell comprises contacting a differentiated adult cell with a Mycobacterium leprae bacterium, or a component thereof selected from the group consisting of a PGL-1, a whole cell wall fraction, a cell wall protein, a cell wall lipid, a cell wall carbohydrate, a protein released from a viable bacterium, and a protein secreted from a viable bacterium.

Patent application WO 2005/068615 provides a method of culturing a pluripotent stem cell while inhibiting differentiation of the pluripotent stem cell by suppression of Wnt/β-catenin signaling. The Wnt/β-catenin signalling suppressor is sFRP, collagen XVIII, endostatin, carboxypeptidase Z, receptor tyrosine kinase, transmembrane enzyme Corin, WIF-1, Cerebus, Dickkopf-1, or a combination thereof.

Patent application WO 2007/016485 discloses the use of a GSK-3 inhibitor to maintain potency of cultured cells. This document is directed to the culture of non-embryonic cells, that can differentiate into cell types of more than one embryonic lineage, in culture under conditions that maintain differentiation capacity during expansion; more particularly, culturing non-embryonic cells in the presence of at least one GKS-3 inhibitor, such as BIO.

Thus, several strategies have been proposed to reprogram differentiated cells such as somatic cell nuclear transplantation, treatment with extracts of pluripotent cells or stable expression of defined factors. However such strategies present several drawbacks including limited application due to availability of oocytes and low cloning efficiency, reprogrammed cell regaining only some of the properties of the pluripotent cells or elicitation of side effects. In addition, reprogramming via treatment with extracts of pluripotent cells leads to very transient, if any, reprogramming of the differentiated cells. Furthermore, the reprogramming via the stable expression of defined factors implies the use of ES-specific factors (Oct4, klf4, sox2, lin28) or one oncogene (myc) which may lead to undesired side effects.

Therefore there is the need to provide a method for reprogramming a somatic cell that display high efficiency of reprogramming and that is more physiological.

### Summary of invention

The present invention relates to a method for reprogramming a differentiated cell to an undifferentiated stem cell, the reprogrammed cell obtained thereby, the use thereof as well as the use of a Wnt/β-catenin activator or of a GSK-3 inhibitor for reprogramming a differentiated cell to an undifferentiated stem cell.

As a matter of facts, in the present invention, the authors show that transient activation of Wnt/β-catenin signaling can trigger reprogramming of three different types of somatic cells. Large numbers of reprogrammed colonies can be generated after fusion of ES cells with NS cells, mouse embryonic fibroblasts or thymocytes, by culturing the hybrids transiently with Wnt3a or a glycogen synthase kinase-3 (GSK-3) inhibitor such as 6-bromoindirubin-3'-oxime (BIO) and CHIR99021. The isolated reprogrammed clones express ES-cell-specific genes, lose somatic differentiation markers, become unmethylated on *Oct4* and *Nanog* CpG islands, and can differentiate into cardiomyoctes *in vitro* and generate teratomas *in vivo.* Then authors identify Wnt/β-catenin as the first pathway that is not constitutively active in ES cells that can kick-off cell reprogramming. This pathway represents a toggle switch for initiation of the cascade of events that leads to reprogramming of somatic cells in living organisms.
Therefore it is an object of the present invention a method for reprogramming a differentiated cell to an undifferentiated stem cell comprising fusing a pluripotent cell with a differentiated cell to form a fused cell, wherein
a) the pluripotent cell is pre-treated or
b) the fused cell is treated
   with a suitable amount of a Wnt/β-catenin pathway activator.

Preferably the pluripotent cell is an adult stem cell. More preferably the pluripotent cell is an embryonic stem cell. Even more preferably the pluripotent cell is a precursor cell.

In a particular embodiment the differentiated cell is a somatic cell. Preferably the differentiated cell is a neural stem cell. More preferably the differentiated cell is an embryonic fibroblast. Even more preferably the differentiated cell is a thymocyte.

In another embodiment the Wnt/β-catenin pathway activator is at least one Wnt protein isoform. Preferably the Wnt protein isoform is selected from the group of: Wnt1, Wnt2, Wnt2b/13, Wnt3, Wnt3a, Wnt4, Wnt5a, Wnt5b, Wnt6, Wnt7a, Wnt8a, Wnt8b, Wnt9a, Wnt9b, Wnt10a, Wnt10b, Wnt11 or Wnt16.

In a preferred embodiment the Wnt/β-catenin pathway activator is a glycogen synthase kinase-3 (GSK3) inhibitor. Preferably, the GSK3 inhibitor is BIO or CHIR99021 or an analogue thereof.

In a still preferred embodiment the method of the invention further comprises overexpressing Nanog protein in the pluripotent cell.

In another preferred embodiment the method of the invention further comprises overexpressing Nanog protein in the fused cell.

Preferably the overexpression of Nanog protein is obtained by genetically modifying the pluripotent cell or the fused cell or by transducing the pluripotent cell or the fused cell with an appropriated vector.

Still preferably the pluripotent cell is a mouse or a human cell and the differentiated cell is a mouse or a human cell.

It is further object of the invention a reprogrammed undifferentiated non embryonic stem cell obtainable according to the method of the invention.

Preferably the reprogrammed undifferentiated non embryonic stem cell is for medical use. More preferably the reprogrammed undifferentiated non embryonic stem cell is for cell therapy.

It is another object of the invention the use of the reprogrammed undifferentiated non embryonic stem cell of the invention for the preparation of a medicament.

In particular the medicament may be used to treat diseases caused by atrophy, necrosis, apoptosis or any other degeneration and subsequent loss of differentiated tissues such as may occur to beta-islets in diabetes, retinal tissues, neural cells and tissues in for instance Parkinson's disease, Alzheimer's disease or Huntington's disease. The medicament may also be used to restore tissues damage through acute or chronic injuries, for instance bone replacement or repair, restoration of myocardial tissue after infarct.

It is a further object of the invention a pharmaceutical composition comprising the reprogrammed undifferentiated non embryonic stem cell obtained according to the method of the invention and suitable excipients and/or diluents and/or carrier.

It is another object of the invention the use of a Wnt/β-catenin pathway activator for reprogramming a differentiated cell to an undifferentiated stem cell. Preferably the Wnt/β-catenin pathway activator is at least one Wnt protein isoform. More preferably, the Wnt protein isoform is selected from the group of: Wnt1, Wnt2, Wnt2b/13, Wnt3, Wnt3a, Wnt4, Wnt5a, Wnt5b, Wnt6, Wnt7a, Wnt8a, Wnt8b, Wnt9a, Wnt9b, Wnt10a, Wnt10b, Wnt11 or Wnt16.

Still preferably the Wnt/β-catenin pathway activator is a glycogen synthase kinase-3 inhibitor.

More preferably the glycogen synthase kinase-3 inhibitor is BIO or CHIR99021 or an analogue thereof.

In the present invention a differentiated cell means a cell specialized for a specific function (such as a heart, liver, or muscle cell) that cannot generate other types of cells. An undifferentiated stem cell is a cell not specialized for a specific function that retains the potential to give rise to specialized cells. A pluripotent cell is a primordial cell that can differentiate into a sub-group of specialized types of cells. An adult stem cell is an undifferentiated cell (called also somatic stem cell) that is present in adult or foetal or new born tissues and is able to differentiate into specialized cells of the tissue from which it originated. An embryonic stem cell is an undifferentiated cell that is present in embryos and is able to differentiate into any type of specialized cell. A precursor cell is a cell that detains a precocious state of differentiation and is already committed to differentiate in cell types of a specific lineage. A somatic cell is any cell of a multicellular organism that will not contribute to gamete cells.

The present invention shall be disclosed in detail in the following description also by means of non limiting examples referring to the following figures.

### Figure 1. Optimization of Wnt3a and BIO concentrations for cell reprogramming.

ES-neo x NS-Oct4-puro fusions treated for 24 h with the indicated Wnt3a (a) or BIO (b) concentrations. After fusion and puromycin selection, the colonies were stained for expression of alkaline phosphatase (AP) and counted. The fold-increases in the number of colonies are shown.

### Figure 2. Schematic overview of the experimental system.

ES-neo cells expressing pluripotent genes were fused with NS-Oct4-puro cells expressing neural specific genes. To test the role of the Wnt/β-catenin pathway in reprogramming by cell fusion, the cells were treated in two ways: (1) ES-neo cells were treated with BIO for different times before being fused with non-treated NS-Oct4-puro cells; or (2) the cells were first fused, and then treated with Wnt3a or BIO for different times. Only following the full reprogramming of the NS-Oct4-puro cell epigenome will colonies of viable primary hybrids of pluripotent cells with an ES cell phenotype form, because: i) the Oct4 promoter will be active in the NS-cell genome and will express the puromycin resistance gene and GFP; and ii) the hybrid cells will proliferate in ES-cell medium (which contains fetal bovine serum and

LIF, two signals that promote differentiation of NS cells).

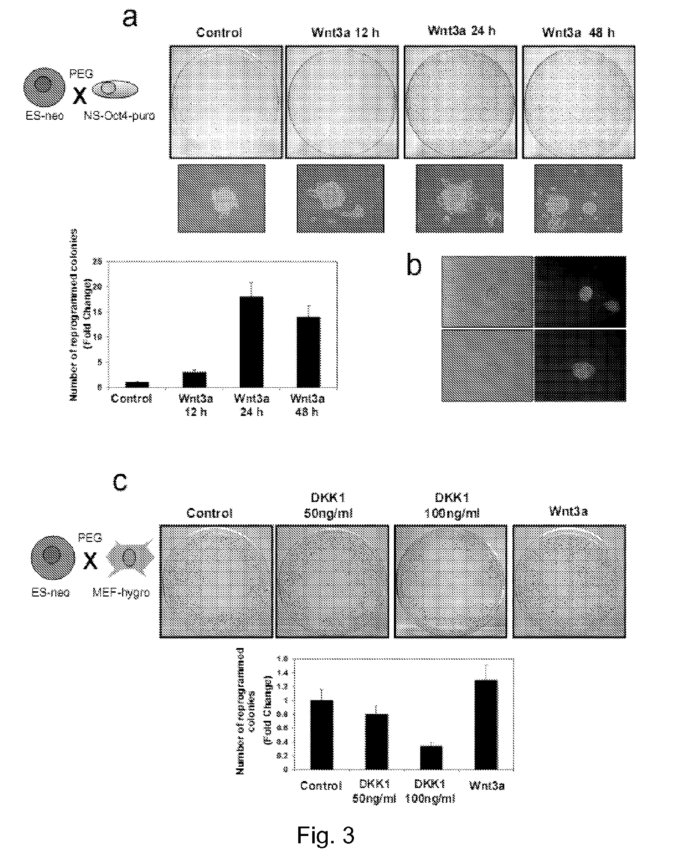

### Figure 3. Activation of the Wnt pathway by Wnt3a enhances reprogramming mediated by cell fusion.

(a) Representative plates and quantification (as fold-increases in the number of colonies) of hybrid colonies from PEG fusions of ES-neo cells with NS-Oct4-puro cells treated with Wnt3a as indicated. Colonies were stained for expression of AP and counted. (b) Fluorescence images of some reprogrammed hybrid colonies following 24 h Wnt3a treatment (10x magnification). (c) Representative plates and quantification of hybrid colonies from PEG fusions of ES-neo cells and MEF-hygro cells treated with DKK1 and Wnt3a as indicated. Colonies were stained for AP expression and counted as above.

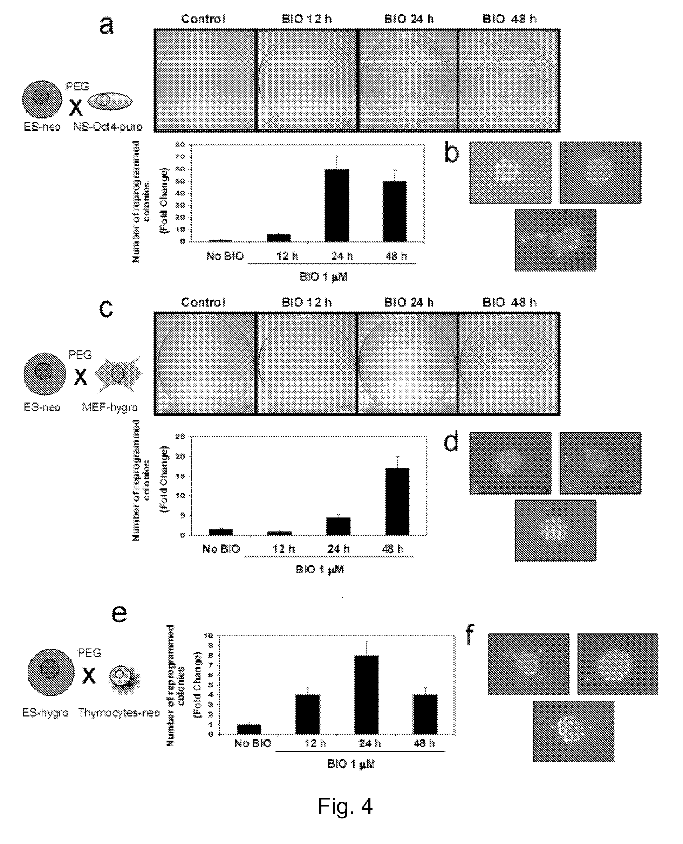

### Figure 4. Activation of the Wnt/β-catenin pathway by BIO enhances the number of reprogrammed hybrid colonies in three different cell-type fusions.

Representative plates (a, c) and quantification (as fold-increases in the number of colonies) (a, c, e) of hybrid colonies from the indicated fusions treated with BIO as indicated, stained for AP expression and counted. The fold-change increases in the reprogrammed colonies are also shown in the graphs. (**b**, **d**, **f**) Light microscopy images of some reprogrammed hybrid colonies following 24 h BIO treatment (10x magnification).

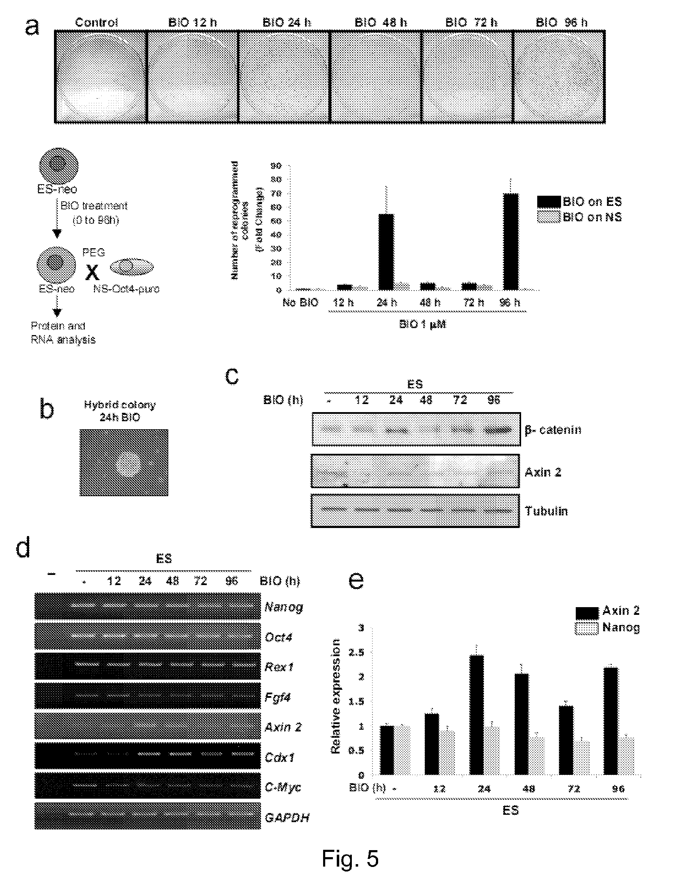

### Figure 5. BIO triggers the Wnt/β-catenin pathway in ES cells and in turn augments the reprogramming of NS cells without activation of stem-cell genes.

(a) Representative plates and quantification (as fold-increases in the number of colonies) of hybrid colonies from PEG fusions between BIO-pretreated ES-Neo cells and non-treated NS-Oct4-puro cells as indicated. Colonies were stained for the expression of AP and counted. The quantification also shows BIO-pretreated NS-Oct4-puro cells fused with non-treated ES-Neo cells (gray bars). (b) Light microscopy image of reprogrammed hybrid colony isolated from fusion of BIO-pretreated (24 h) ES-neo cells and NS-Oct4-puro cells (10x magnification). (c) Western blotting of protein extracts from ES cells from the BIO-pretreatments in (a), as indicated. (d) Semi-quantitative RT-PCR analysis of ES-cell-specific genes (*Nanog, Oct4, Rex1* and *Fgf4*) and β-catenin targets (*Axin2, Cdx1* and *c-Myc*) in total mRNA purified from ES cells from the BIO-pretreatments in (a), as indicated. (e) Quantitative RT-PCR analysis of *Axin2* and *Nanog* transcript levels in ES cells from the BIO-pretreatments in (a), as indicated.

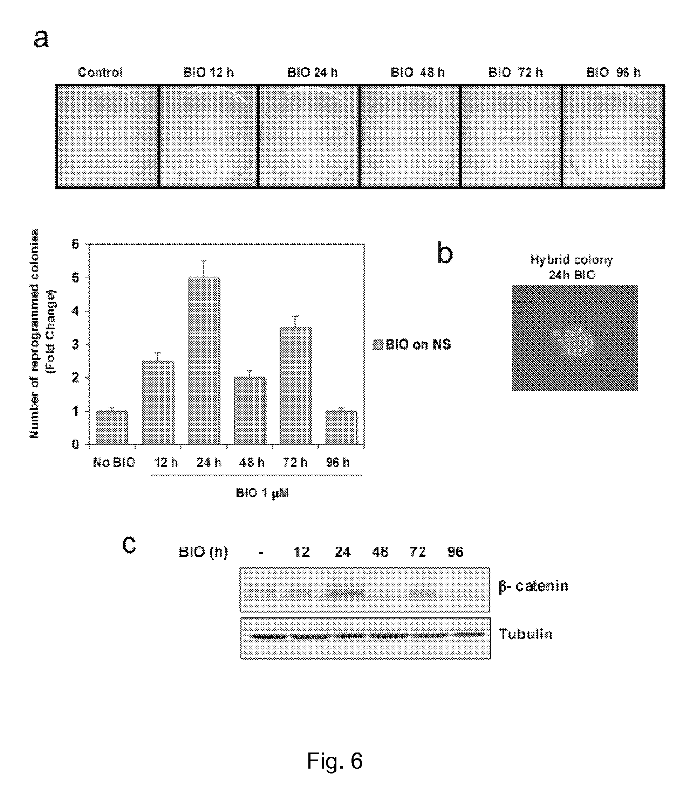

### Figure 6. Treatment of NS-Oct4-puro cells with BIO before their fusion with ES-neo cells results in poor enhancement of reprogramming.

**(A)** Plates containing fused hybrid colonies between BIO-treated NS-Oct4-puro cells and non treated ES-neo cells. The clones were stained for the expression of AP and counted. The fold change increases in the reprogrammed colonies are shown in the graphs. (B) Example of a reprogrammed colony isolated from ES neo cells fused with NS-Oct4-puro cells pretreated with BIO for 24h (C) Western blotting of total protein extracts of the NS-Oct4-puro cells at the indicated times.

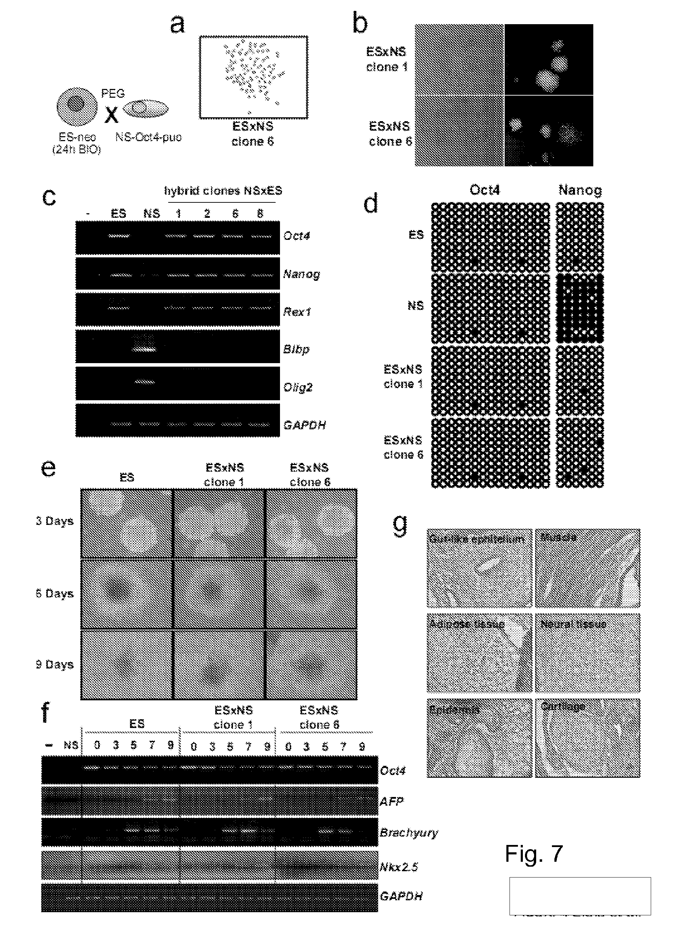

### Figure 7. Isolated hybrids are pluripotent and can differentiate in vitro and in vivo.

Reprogrammed clones isolated from fusions of of BIO-pretreated (24 h) ES-neo cells and NS-Oct4-puro cells. **(a)** normal karyotype and **(b)** GFP expression in the different reprogrammed hybrid clones. (c) RT-PCR analysis of *Oct4, Nanog, Rex1, Blbp* and *Olig2* of ES cells, NS cells and some of the hybrid clones, as indicated. **(d)** Bisulfite genomic sequencing of the promoter regions of *Oct4* and *Nanog* in ES cells, NS cells and hybrid clones. Open circles indicate non-methylated CpG dinucleotides; closed circles indicate methylated CpGs. (**e**) Morphology of embryoid bodies during differentiation, as indicated. (**f**) RT-PCR analysis of differentiation markers in ES cells, NS cells and hybrid clones, as indicated. (**g**) Hematoxylin and eosin staining of teratoma derived from cells of one reprogrammed clone transplanted subcutaneously into SCID mice.

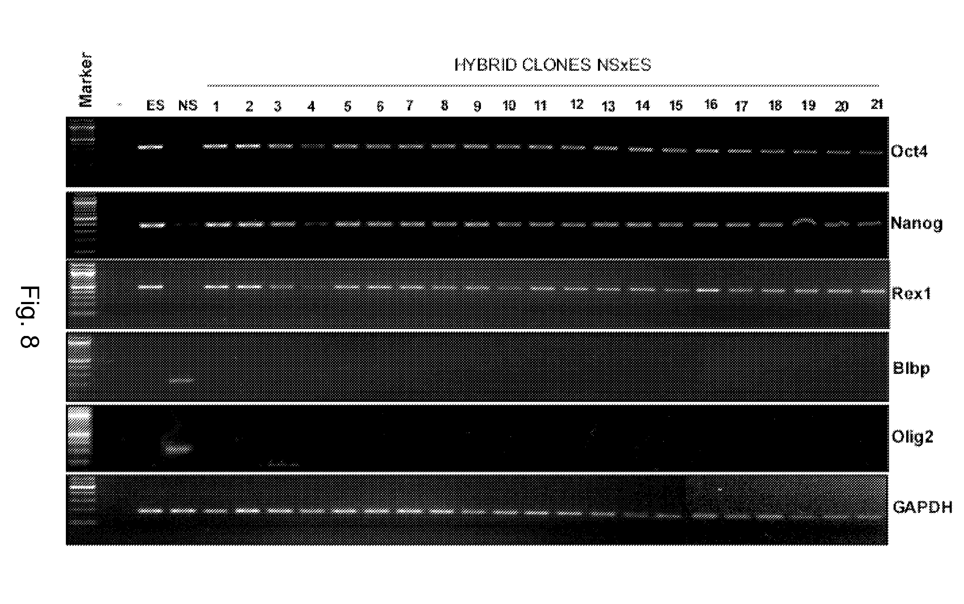

### Figure 8. Analysis of 21 different hybrid clones isolated from the fusion of NS-Oct4-puro cells with ES -neo cells that were pre-treated with BIO.

RT-PCR analysis of *Oct4, Nanog, Rex1, Blbp* and *Olig2* in ES-Neo, in NS-Oct4-puro and in 21 hybrid clones.

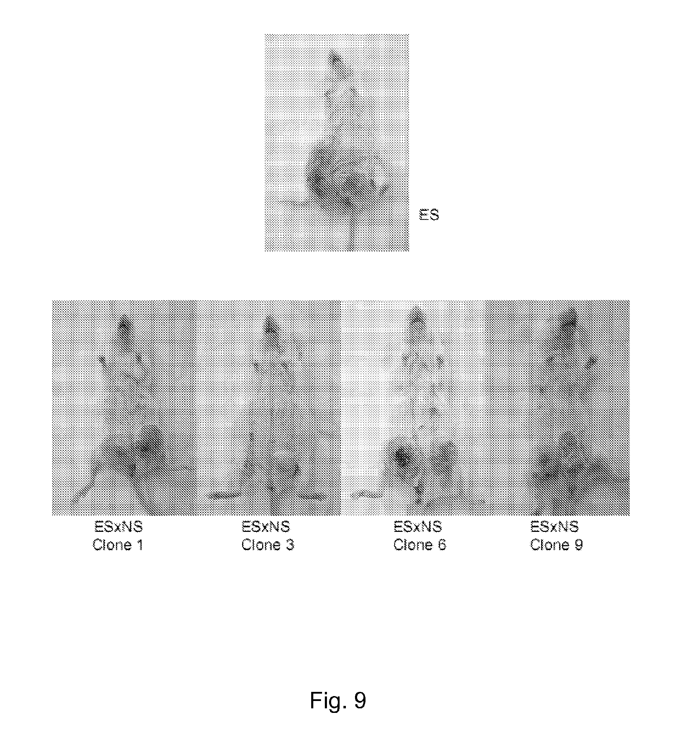

### Figure 9. Teratoma formation of ES-neoxNS-Oct4-puro hybrid clones.

1,5x10⁷ hybrid cells were injected subcutaneously in immuno-deficient (SCID) mice. Four weeks after injection, teratomas were photographed before to be collected and analyzed.

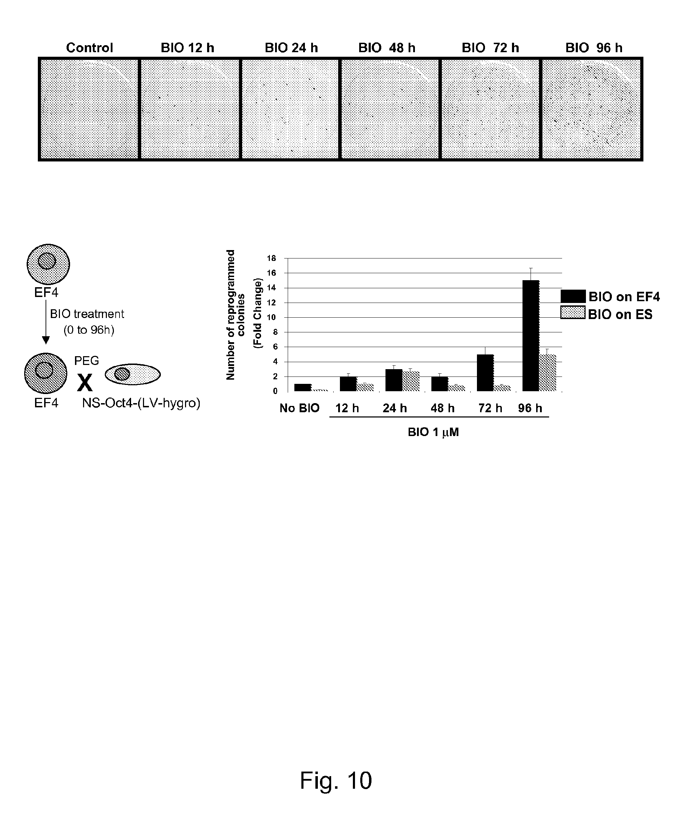

### Figure 10. Nanog over-expression and Wnt pathway activation cooperate to enhance reprogramming of NS cells.

Representative plates and quantification (as fold-increases in the number of colonies) of hybrid colonies from PEG fusions between BIO-pretreated EF4 cells and non-treated NS-Oct4-(LV-Hygro) cells as indicated. Colonies were stained with giemsa and counted. The quantification also shows BIO-pretreated ES-Hygro cells fused with non-treated NS-Oct4-Puro cells (gray bars).

### Methods

### Cells

NS-Oct4-puro cells isolated from HP165 mice and carrying the regulatory sequences of the mouse *Oct4* gene driving GFP and puromycin resistance genes were a gift of Dr. A. Smith and were cultured as previously described ²². Thymocytes were isolated from 6-8-week-old mice. Hygromycin-resistant MEFs were purchased at passage 3 (Millipore). ES-neo and ES-hygro cells were derived from E14Tg2a and transduced with the lentiviral pHRcPPT-PGK-Neomycin and pHRcPPT-PGK-Hygromycin vectors ²³. ES cells were cultured on gelatin in Knock-out Dulbecco's modified Eagle's medium (DMEM) supplemented with 20% fetal bovine serum (Hyclone), 1x non-essential amino acids, 1x GlutaMax, 1x 2-mercaptoethanol and 1,000 U/ml LIF ESGRO (Chemicon).

### Cell hybrids

NS x ES and MEF x ES PEG fusions: 1.2 x10⁶ NS cells or MEFs were plated into a T25 flask. After 2 h, 1.2 x10⁶ ES cells were plated onto them, allowing them to attach to the NS cells or MEFs. After a further 2 h, 1 ml 50% (w/v) PEG 1450 (Sigma; pre-warmed to 37 °C) was added for 2 min, and then the cells were washed three times with serum-free DMEM.

Finally, they were cultivated with ES-cell complete medium without and with 1 µM BIO (Calbiochem), CHIR99021 (6-{2-[4-(2,4-dichlorophenyl)-5-(4-methyl-1H-imidazol-2-yl)-pyrimidin-2-ylamino]ethylamino}nicotinonitrile)²⁴, 100 ng/ml Wnt3a (R&D) or 50-100 ng/ml DKK1 (R&D). After 12 h, the cells were trypsinized and plated into gelatin+laminin-treated p100 dishes. For the NS x ES selection, puromycin was added after 72 h, while for the MEF x ES selection, hygromycin plus neomycin were added after 24 h.

Thymocyte x ES fusion: the cells were fused in suspensions as previously described ⁴. After fusion, they were packed by centrifugation and the supernatant was discarded. The pelleted cells were re-suspended in complete ES-cell medium and plated. The selection was carried out after 24 h, using hygromycin plus neomycin.

### Western blotting

Western blotting was performed as previously described ²³. The primary antibodies used were anti-β-catenin, clone 14 (BD Biosciences); anti-conductin (axin 2), sc-20784 (Santa Cruz); anti-APC, sc-895 (Santa Cruz); and anti-β-tubulin, clone D66, T0198 (Sigma).

### Semiquantitative RT-PCR analysis

For RT-PCR, the total RNA was extracted from ES cells and from embryoid bodies (200 embryoid bodies for each clone and for each differentiation time point) using the RNeasy Kit (Qiagen), and the cDNA was generated using superscript III (Invitrogen). The primers used were:

| | |
|---|---|
| Oct4: | forward, GGCGTTCTCTTTGGAAAGGTGTTC (SEQ ID No. 1); |
| | reverse, CTCGAACCACATCCTTCTCT (SEQ ID No. 2). |
| Nanog: | forward, AGGGTCTGCTACTGAGATGCTCTG (SEQ ID No. 3); |
| | reverse, CAACCACTGGTTTTTCTGCCACCG (SEQ ID No. 4). |
| Rex1: | forward, GCCCTCGACAGACTGACCCTAA (SEQ ID No. 5); |
| | reverse, CTTCCTCAGGGCGGTTTTACCC (SEQ ID No. 6). |
| Fgf4: | forward, GACTACCTGCTGGGCCTCAA (SEQ ID No. 7); |
| | reverse, CGACACTCGGTTCCCCTTCT (SEQ ID No. 8). |
| Olig2: | forward, GCGTGGGTATCAGAAGCACT (SEQ ID No. 9); |
| | reverse, CCAGTCGGGTAAGAAACCAA (SEQ ID No. 10). |
| Blbp: | forward, GGGTAAGACCCGAGTTCCTC (SEQ ID No. 11); |
| | reverse, ATCACCACTTTGCCACCTTC (SEQ ID No. 12). |
| Axin2: | forward GGGAGCAGTTTTGTGGCAGCA (SEQ ID No. 13); |
| | reverse, AGGGTCCTGGGTAAATGGGTGAG (SEQ ID No. 14) |
| C-Myc: | forward, TGCCGCCCACTCTCCCCAACC (SEQ ID No. 15); |
| | reverse, CCGCCGCCGTCATCGTCTTCC (SEQ ID No. 16). |
| Brachyury: | forward, TGCTGCCTGTGAGTCATA (SEQ ID No. 17); |
| | reverse, ACAAGAGGCTGTAGAACATG (SEQ ID No. 18). |
| Nkx2.5: | forward, GCTCTCCTGCTTTCCCAGC (SEQ ID No. 19); |
| | reverse, CTCCCATCCCTACTGCCTTCTGCAGC (SEQ ID No. 20). |
| AFP: | forward, TCCCTCATCCTCCTGCTA (SEQ ID No. 21); |
| | reverse, GCACATTCTTCTCCGTCAC (SEQ ID No. 22). |
| Cdx1: | forward TCTACACAGACCACCAACGC (SEQ ID No. 23) |
| | reverse AGAAACTCCTCCTTGACGGG (SEQ ID No. 24). |
| FGF5: | forward, AAAGTCAATGGCTCCCACGAA (SEQ ID No. 25); |
| | reverse, CTTCAGTCTGTACTTCACTGG (SEQ ID No. 26). |
| GAPDH: | forward, ACTCCCACTCTTCCACCTTC (SEQ ID No. 27); |
| | reverse, TCTTGCTCAGTGTCCTTGC (SEQ ID No. 28). |

### Bisulfite genomic sequencing

Bisulfite treatment was performed using the Epitect Bisulfite Kit (Qiagen), according to the manufacturer recommendations. The amplified products were cloned into pCR2.1-TOPO (Invitrogen). Ten randomly selected clones were sequenced with the M13 forward and M13 reverse primers for each gene, as follows:

| | |
|---|---|
| MeNanog: | forward, GATTTTGTAGGTGGGATTAATTGTGAATTT (SEQ ID No. 29); |
| | reverse, ACCAAAAAAACCCACACTCATATCAATATA (SEQ ID No. 30). |
| MeOct4: | forward, GGTTTTTTAGAGGATGGTTGAGTG (SEQ ID No. 31); |
| | reverse, TCCAACCCTACTAACCCATCACC (SEQ ID No. 32). |

### Quantitative RT-PCR

RNA isolation and reverse transcription were carried out as described for semiquantitative RT-PCR. The template for each PCR reaction was the cDNA obtained from 16 ng total RNA in a 25-µl reaction volume. Platinum SYBR green qPCix-UDG (Invitrogen) was used with an ABprism 7000 real-time PCR machine, according to the manufacturer recommendations. The primers used were:

| | |
|---|---|
| NanogRT: | forward, AACCAAAGGATGAAGTGCAAGCGG (SEQ ID No. 33); |
| | reverse, TCCAAGTTGGGTTGGTCCAAGTCT (SEQ ID No. 34). |

Axin2 (purchased, SuperArray Bioscience Corporation).

### In-vitro differentiation of hybrid cells

The differentiation medium for the production of embryoid bodies consisted of ES-cell maintenance medium with no LIF supplementation. The cells were harvested by trypsinization, counted and propagated in hanging drops (400 single ES cells/30 µl initial drop) for 2 days before being transferred to 10 cm² bacterial dishes. At day 5, the embryoid bodies were transferred onto gelatinized p100 dishes.

### Teratoma production

Cells were trypsinized into single-cell suspensions and resuspended in phosphate-buffered saline to a concentration of 1.5 x10⁷ cells/ml. These cells were injected subcutaneously into the hind limbs of Fox Chase SCID mice using a 25-guage needle (200 µl). Teratomas were collected after 4 weeks, and were fixed, embedded, sectioned and stained.

### Nanog expression

### Cells

NS-Oct4-(LV-Hygro) cells were derived from NS-Oct4-puro and transduced with the letiviral pHRcPPT-PGK-Hygromycin vector. EF4 cells carrying the CAG promoter driving Nanog and Puromycin resistance genes were a gift of Dr. I. Chambers. Cells were cultured as previously described²⁵.

### Cell hybrids

NSxES-Hygro or NS-HygroxEF4 cells PEG fusions were done as previously described. For the NSxES-Hygro and NSxEF4 selection, Puromycin plus Hygromycin was added after 72 h.

### Results

The binding of Wnt ligands to the Frizzled receptor results in inactivation of a multiprotein complex composed of glycogene synthase kinase-3 (GSK-3), Axin1, Axin2 and adenomatous polyposis coli (APC). Inactivation of GSK-3 leads to a decrease in β-catenin phosphorylation, which then escapes ubiquitin/ proteosome-mediated degradation ⁸. As a consequence, non-degraded β-catenin accumulates in the cytoplasm and translocates into the nucleus, leading to transcriptional activation of a plethora of target genes ⁹. Wnt3a-cultured embryonic stem (ES) cells can self renew and remain totipotent due to the accumulation of β-catenin ¹⁰. In addition, both inactivation of the *GSK-3*α and *GSK-3*β homologs and mutations in *APC* severely compromise the ability of ES cells to differentiate ^{11, 12}. Of note, the regeneration of hair follicles and of cardiovascular progenitors is Wnt dependent ^{11, 14}. These observations prompted the authors to determine if Wnt/β-catenin signaling is a pathway that can drive reprogramming of somatic cells.

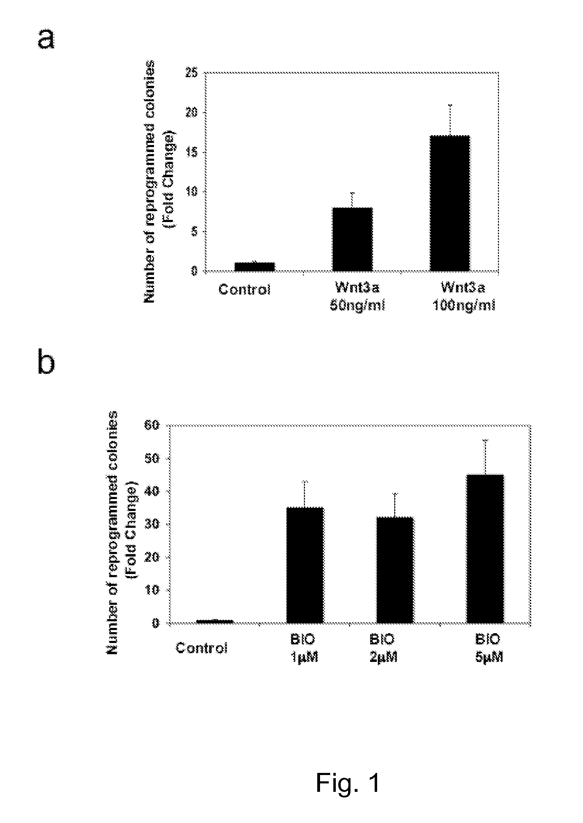

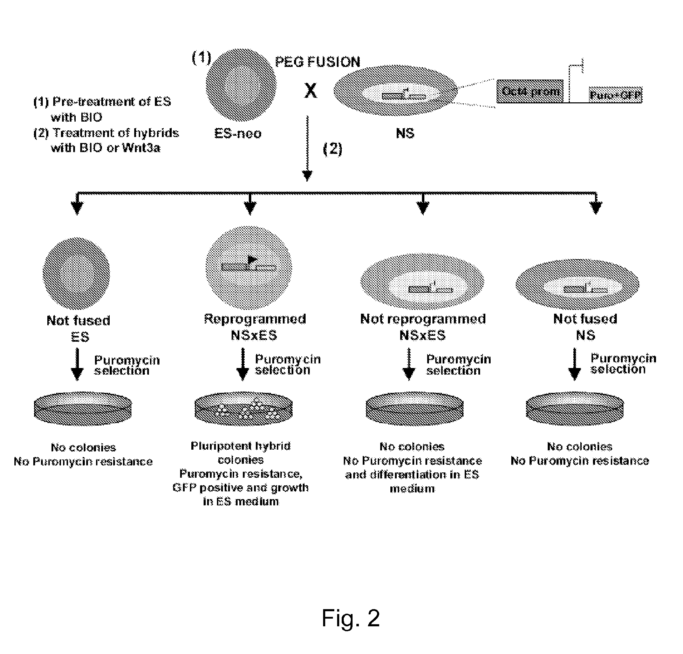

To test this hypothesis, the authors used polyethyleneglycol (PEG) to generate hybrids between ES cells constitutively expressing the *NeoR* gene, and mouse neural stem (NS)-Oct4-puro cells ⁴ expressing the *PuroR* gene under control of the *Oct4* (*Pou5f1*) regulatory element that is only active in pluripotent cells ¹⁵. Immediately after fusion, the cells were cultured in gelatin (without feeders that express Wnt) for 12, 24 and 48 h in the presence of an optimized concentration of Wnt3a (100 ng/ml; Fig. 1a). Hybrids were selected in ES-cell medium supplemented with puromycin, where only reprogrammed clones can grow (Fig. 2). These resistant colonies were stained for expression of alkaline phosphatase (AP), an ES-cell marker, and counted (Fig. 3a). These cells had been reprogrammed, as they retained rounded ES-cell-like phenotype and expressed AP, Oct4-puro, and green fluorescent protein (GFP; the NS-Oct4-puro cells express puromycin/ GFP-fused coding regions) (Fig. 3a, b). The authors selected up to 20-fold more reprogrammed clones with respect to the control. These data clearly showed that Wnt3a greatly enhanced the ability of ES cells to reprogram NS cells.

In support of these data, a very high number of reprogrammed clones (between 30 and 300) were obtained when mouse embryonic fibroblasts (MEFs) expressing different Wnt isoforms ¹⁶ were fused with ES cells (Fig. 3c). As expected, the number of reprogrammed clones did not increase greatly when these hybrids were cultured in the presence of Wnt3a, as MEFs already produce sufficient amounts of Wnt (Fig. 3c).

Finally, to confirm that activation of the Wnt/β-catenin pathway enhances somatic cell reprogramming, the authors inhibited it with DKK1 ¹⁷. The striking decrease in the number of reprogrammed, AP-positive clones (up to 3-fold; Fig. 3c) demonstrated that the Wnt/β-catenin signaling pathway is a toggle switch for the reprogramming of somatic cells.

To confirm this further, the authors used a different approach. PEG-fused ES-neo x NS-Oct4-puro cells were cultured for 12, 24 and 48 h with an optimized concentration of the GSK-3 inhibitor 6-bromoindirubin-3'-oxime (BIO; 1 µM; Fig 4a and Fig. 1b), which promotes accumulation of β-catenin by inhibition of GSK-3 ¹⁸ and thus maintains the pluripotency of stem cells ¹⁶. After 24 and 48 h of BIO treatment, the authors obtained up to 70-fold more reprogrammed clones (from 200 to 1,500 total clones) with respect to the control (Fig. 4a). The selected hybrids showed an ES-like phenotype, were AP-positive, and expressed Oct4-puro (Fig. 4b).

Inhibition of GSK-3 with BIO also strikingly increased the numbers of reprogrammed colonies when MEFs and thymocytes were fused with ES cells. PEG-fused hybrids of ES-neo x MEF-hygro and ES-hygro x thymocyte-neo cells were cultured in BIO for 12, 24 and 48 h and double-drug selected. There were up to 20-fold (between 200 and 1,500 total clones) and 9-fold (between 20 and 100 total clones) increases in the numbers of reprogrammed selected clones, with respect to their controls (fusions in the absence of BIO), respectively (Fig. 4c-e). Again, these clones showed an ES-like phenotype and were AP positive (Fig. 4c-f). These data demonstrate that transient inhibition of GSK-3 greatly enhances the ability of ES cells to reprogram somatic cells. Furthermore, this reprogramming occurs with variable timing, as the increases in reprogramming were different across the various cell types.

The authors then determined whether enhancement of reprogramming can occur when ES and/or NS cells were cultured with BIO before their fusion. Here, ES cells were cultured in BIO for 12, 24, 48, 72 and 96 h, and then fused with NS-Oct4-puro cells grown in the absence of BIO. Up to an 80-fold increase (about 2,000 total clones) in the number of reprogrammed clones was seen when ES cells were pre-cultured for 24 or 96 h in BIO (Fig. 5a); the selected clones showed an ES-like phenotype, were AP-positive, and expressed *Oct4* (Fig. 5a, b). In contrast, there was poor enhancement of NS reprogramming with the ES cells cultured with BIO for 12, 48 and 72 h; the authors thus wondered why there was a parabolic wave of reprogramming through the period of this BIO treatment. While 12 h of BIO treatment was not sufficient to accumulate enough β-catenin to trigger reprogramming, 24 h (and 96 h) of BIO pretreatment of ES cells resulted in their accumulation of β-catenin; at the same time points, *Axin2* and *APC* were also expressed, leading to a negative feedback loop on the pathway ¹¹ that affected the subsequent 48 h and 72 h time points (Fig. 5c, and data not shown). In conclusion, only when β-catenin accumulates in ES cells can these reprogram NS cells after fusion.

Remarkably, BIO-treated ES cells expressed the same levels of the pluripotent genes *Nanog, Oct4, Rexl* and *Fgf4* at all treatment times, whereas expression of the β-catenin-dependent genes *Cdx1* and *Axin2* cycled according to accumulation of the β-catenin protein itself (Fig. 5d, e). Of note, although *c-Myc* has previously been shown to enhance reprogramming when over-expressed together with three other genes ⁵, here it was constantly expressed under BIO treatment. These data suggest that pretreatment of ES cells with BIO results in expression of specific β-catenin target gene(s) that, in turn, kick-off reprogramming of somatic cells. This pathway is independent of *Nanog, Oct4, Rex1, Fgf4* and *c-Myc;* indeed, these genes were expressed at the same levels during the different BIO treatments. The alternative of pretreatment ofNS cells with BIO led to β-catenin accumulation at 24 h and 72 h, but provided poor enhancement of reprogramming, with only a slight increase in the number of ES-cell-like, AP-positive reprogrammed clones (Fig. 5a and Fig. 6). Thus, the Wnt/β-catenin signaling pathway drives a remarkable enhancement of reprogramming of somatic cells only when activated in ES cells.

To confirm the ES-like phenotype, 21 different reprogrammed clones were isolated from the fusion of ES cells pre-treated with BIO for 24 h and NS-Oct4-puro cells. The clones were all tetraploids (Fig. 7a), were GFP-positive, and expressed *Oct4, Nanog* and *Rex1* after several passages. In contrast, the neural *Blbp* and *Olig2* genes were shut off (Fig. 7b, c and

Fig. 8). Furthermore, bisulfite genomic sequence analysis revealed that in the reprogrammed clones, the CpG islands of the *Nanog* and *Oct4* promoters were highly unmethylated and were comparable to ES cells; in contrast, the same CpGs were highly methylated in parental NS cells (Fig. 7d). These data clearly show that hybrids isolated from these fusions with ES cells pre-treated with BIO had been reprogrammed.

Next, to investigate the differentiation ability of the reprogrammed clones, the authors induced seven clones to spontaneous differentiation. Embryoid bodies were formed; ball-shaped structures were clearly visible after three days and underwent differentiation after 6 and 9 days (Fig 7e). Expression of *Oct4* (primitive ectoderm), *AFP* (endoderm), *Brachyury* (mesoderm) and *Nkx2.5* (cardiac muscle) were seen at 3, 5, 7 and 9 days of differentiation, with the expected timing ¹⁹, and comparable to ES cells (Fig. 7f). Furthermore, some of the clones started beating spontaneously, demonstrating their differentiation into cardiomyocytes. Finally, to test the differentiation ability of these cells *in vivo,* the authors transplanted five different clones subcutaneously in immuno-deficient (SCID) mice. Three weeks after injection, teratomas were seen (Fig. 9). Histological examination showed the presence of gut-like epithelial tissue (endoderm), striated muscle and adipose tissue (mesoderm), neural tissue, epidermis, and cartilage (ectoderm) (Fig. 7g). These data clearly demonstrated the differentiation capacity of the reprogrammed clones, both *in vitro* and *in vivo.*

The authors' data demonstrate that activation of the Wnt/β-catenin signaling pathway without over-expression of any of the known ES-cell genes can control reprogramming of somatic cells. Indeed, in a system with both Nanog overexpression and Wnt pathway activation, the reprogramming of NS cells is enhanced even further (Fig. 10), indicating that these two pathways can cooperate, but remain distinct. The authors have shown that the cycling accumulation of β-catenin determines reprogramming, mimicking a physiological scenario that might occur *in vivo.* In contrast, stable gain-of-function of β-catenin may result in tumor formation ²⁰ or the inability of reprogrammed cells to differentiate after fusion ^{11, 12}, whereas its loss of function may result in failure of reprogramming ²¹.

Finally, the authors postulate that a novel gene that is probably an epigenetic factor that is not constitutively expressed in ES cells can be activated via Wnt/β-catenin signaling, to switch on a cascade of events that triggers reprogramming of the genome of somatic nuclei. Our data lead us to the highly speculative and provocative hypothesis that regeneration of injured tissues might be kicked off via "commanding" stem cells that are activated via the

Wnt/β-catenin pathway and that are able to reprogram somatic cells after fusion.

### References

1. Tada, M., Tada, T., Lefebvre, L., Barton, S. C. & Surani, M. A. Embryonic germ cells induce epigenetic reprogramming of somatic nucleus in hybrid cells. EMBO J 16, 6510-20 (1997).
2. Tada, M., Takahama, Y., Abe, K., Nakatsuji, N. & Tada, T. Nuclear reprogramming of somatic cells by in-vitro hybridization with ES cells. Curr Biol 11, 1553-8 (2001).
3. Cowan, C. A., Atienza, J., Melton, D. A. & Eggan, K. Nuclear reprogramming of somatic cells after fusion with human embryonic stem cells. Science 309, 1369-73 (2005).
4. Silva, J., Chambers, I., Pollard, S. & Smith, A. Nanog promotes transfer of pluripotency after cell fusion. Nature 441, 997-1001 (2006).
5. Takahashi, K. & Yamanaka, S. Induction of pluripotent stem cells from mouse embryonic and adult fibroblast cultures by defined factors. Cell 126, 663-76 (2006).
6. Takahashi, K. et al. Induction of pluripotent stem cells from adult human fibroblasts by defined factors. Cell (2007).
7. Yu, J. et al. Induced pluripotent stem cell lines derived from human somatic cells. Science (2007).
8. Willert, K. & Jones, K. A. Wnt signaling: is the party in the nucleus? Genes Dev 20, 1394-404 (2006).
9. Hoppler, S. & Kavanagh, C. L. Wnt signalling: variety at the core. J Cell Sci 120, 385-93 (2007).
10. Anton, R., Kestler, H. A. & Kuhl, M. Beta-catenin signaling contributes to stemness and regulates early differentiation in murine embryonic stem cells. FEBS Lett 581, 5247-54 (2007).
11. Doble, B. W., Patel, S., Wood, G. A., Kockeritz, L. K. & Woodgett, J. R. Functional redundancy of GSK-3alpha and GSK-3beta in Wnt/beta-catenin signaling shown by using an allelic series of embryonic stem cell lines. Dev Cell 12, 957-71 (2007).
12. Kielman, M. F. et al. APC modulates embryonic stem-cell differentiation by controlling the dosage of beta-catenin signaling. Nat Genet 32, 594-605 (2002).
13. Ito, M. et al. Wnt-dependent de-novo hair follicle regeneration in adult mouse skin after wounding. Nature 447, 316-20 (2007).
14. Qyang, Y. et al. The renewal and differentiation of Isl1+ cardiovascular progenitors are controlled by a Wnt/B-catenin pathway. Cell Stem Cell (2007).
15. Yeom, Y. I. et al. Germline regulatory element of Oct-4 specific for the totipotent cycle of embryonal cells. Development 122, 881-94 (1996).
16. Sato, N., Meijer, L., Skaltsounis, L., Greengard, P. & Brivanlou, A. H. Maintenance of pluripotency in human and mouse embryonic stem cells through activation of Wnt signaling by a pharmacological GSK-3-specific inhibitor. Nat Med 10, 55-63 (2004).
17. Logan, C. Y. & Nusse, R. The Wnt signaling pathway in development and disease. Annu Rev Cell Dev Biol 20, 781-810 (2004).
18. Meijer, L. et al. GSK-3-selective inhibitors derived from Tyrian purple indirubins. Chem Biol 10, 1255-66 (2003).
19. Boheler, K. R. et al. Differentiation of pluripotent embryonic stem cells into cardiomyocytes. Circ Res 91, 189-201 (2002).
20. Katoh, M. & Katoh, M. WNT signaling pathway and stem cell signaling network. Clin Cancer Res 13, 4042-5 (2007).
21. Haegel, H. et al. Lack of beta-catenin affects mouse development at gastrulation. Development 121, 3529-37 (1995).
22. Conti, L. et al. Niche-independent symmetrical self-renewal of a mammalian tissue stem cell. PLoS Biol 3, e283 (2005).
23. Zito, E. et al. Sulphatase activities are regulated by the interaction of sulphatase-modifying factor 1 with SUMF2. EMBO Rep 6, 655-60 (2005).
24. Meijer L, Flajolet M, Greengard P. Pharmacological inhibitors of glycogen synthase kinase 3. Trends Pharmacol Sci. 25(9):471-80 (2004)
25. Chambers I, Colby D, Robertson M, Nichols J, Lee S, Tweedie S, Smith A. Functional expression cloning of Nanog, a pluripotency sustaining factor in embryonic stem cells. Cell 113(5):643-55 (2003).

## Claims

1. A method for reprogramming a differentiated cell to an undifferentiated stem cell comprising fusing a pluripotent cell with a differentiated cell to form a fused cell, wherein
a) the pluripotent cell is pre-treated or
b) the fused cell is treated
with a suitable amount of a Wnt/β-catenin pathway activator.

2. The method according to claim 1 wherein the pluripotent cell is an adult stem cell.

3. The method according to claim 1 wherein the pluripotent cell is an embryonic stem cell.

4. The method according to claim 1 wherein the pluripotent cell is a precursor cell.

5. The method according to claim 1 wherein the differentiated cell is a somatic cell.

6. The method according to claim 1 wherein the differentiated cell is a neural stem cell.

7. The method according to claim 1 wherein the differentiated cell is an embryonic fibroblast.

8. The method according to claim 1 wherein the differentiated cell is a thymocyte.

9. The method according to any of previous claims wherein the Wnt/β-catenin pathway activator is at least one Wnt protein isoform.

10. The method according to claim 9 wherein the Wnt protein isoform is selected from the group of: Wnt1, Wnt2, Wnt2b/13, Wnt3, Wnt3a, Wnt4, Wnt5a, Wnt5b, Wnt6, Wnt7a, Wnt8a, Wnt8b, Wnt9a, Wnt9b, Wnt10a, Wnt10b, Wnt11 or Wnt16.

11. The method according to any of claims from 1 to 8 wherein the Wnt/β-catenin pathway activator is a glycogen synthase kinase-3 inhibitor.

12. The method according to claim 11 wherein the glycogen synthase kinase-3 inhibitor is BIO or CHIR99021 or an analogue thereof.

13. The method according to claim 1 further comprising overexpressing Nanog protein in the pluripotent cell.

14. The method according to claim 1 further comprising overexpressing Nanog protein in the fused cell.

15. The method according to claim 13 or 14 wherein the overexpression of Nanog protein is obtained by genetically modifying the pluripotent cell or the fused cell or by transducing the pluripotent cell or the fused cell with an appropriated vector.

16. The method according to any of previous claims wherein the pluripotent cell is a mouse or a human cell and the differentiated cell is a mouse or a human cell.

17. A reprogrammed undifferentiated non embryonic stem cell obtainable according to the method of any of previous claims.

18. The reprogrammed undifferentiated non embryonic stem cell according to claim 17 for medical use.

19. The reprogrammed undifferentiated non embryonic stem cell according to claim 17 for cell therapy.

20. Use of the reprogrammed undifferentiated non embryonic stem cell according to claim 17 for the preparation of a medicament.

21. A pharmaceutical composition comprising the reprogrammed undifferentiated non embryonic stem cell obtained according to the method of any of claims 1-16 and suitable excipients and/or diluents and/or carrier.

22. Use of a Wnt/β-catenin pathway activator for reprogramming a differentiated cell to an undifferentiated stem cell.

23. The use according to claim 22 wherein the Wnt/β-catenin pathway activator is at least one Wnt protein isoform.

24. The use according to claim 23 wherein the Wnt protein isoform is selected from the group of: Wnt1, Wnt2, Wnt2b/13, Wnt3, Wnt3a, Wnt4, Wnt5a, Wnt5b, Wnt6, Wnt7a, Wnt8a, Wnt8b, Wnt9a, Wnt9b, Wnt10a, Wnt10b, Wnt11 or Wnt16.

25. The use according to claim 22 wherein the Wnt/β-catenin pathway activator is a glycogen synthase kinase-3 inhibitor.

26. The use according to claim 25 wherein the glycogen synthase kinase-3 inhibitor is BIO or CHIR99021 or an analogue thereof.


SEQUENCE LISTING

<110>  Fondazione Telethon
       Cosma, Maria Pia
       Lluis Vinas, Frederic

<120>  Method for reprogramming differentiated cells

<130>  BE 101669

<160>  34

<170>  PatentIn version 3.3

<210>  1
<211>  24
<212>  DNA
<213>  Artificial

<220>
<223>  Synthetic primer

<400>  1
ggcgttctct ttggaaaggt gttc                                              24


<210>  2
<211>  20
<212>  DNA
<213>  Artificial

<220>
<223>  Synthetic primer

<400>  2
ctcgaaccac atccttctct                                                   20


<210>  3
<211>  24
<212>  DNA
<213>  Artificial

<220>
<223>  Synthetic primer

<400>  3
agggtctgct actgagatgc tctg                                              24


<210>  4
<211>  24
<212>  DNA
<213>  Artificial

<220>
<223>  Synthetic primer

<400>  4
caaccactgg tttttctgcc accg                                              24


<210>  5
<211>  22
<212>  DNA
<213>  Artificial

<220>
<223>  Synthetic primer

<400>  5
gccctcgaca gactgaccct aa                                                22

<210>  6
<211>  22
<212>  DNA
<213>  Artificial

<220>
<223>  Synthetic primer

<400>  6
cttcctcagg gcggttttac cc                                                22

<210>  7
<211>  20
<212>  DNA
<213>  Artificial

<220>
<223>  Synthetic primer

<400>  7
gactacctgc tgggcctcaa                                                   20

<210>  8
<211>  20
<212>  DNA
<213>  Artificial

<220>
<223>  Synthetic primer

<400>  8
cgacactcgg ttccccttct                                                   20

<210>  9
<211>  20
<212>  DNA
<213>  Artificial

<220>
<223>  Synthetic primer

<400>  9
gcgtgggtat cagaagcact                                                   20

<210>  10
<211>  20
<212>  DNA
<213>  Artificial

<220>
<223>  Synthetic primer

<400>  10
ccagtcgggt aagaaaccaa                                                   20

<210>  11
<211>  20
<212>  DNA
<213>  Artificial

<220>
<223>  Synthetic primer

<400>  11
gggtaagacc cgagttcctc                                                    20


<210>  12
<211>  20
<212>  DNA
<213>  Artificial

<220>
<223>  Synthetic primer

<400>  12
atcaccactt tgccaccttc                                                    20


<210>  13
<211>  21
<212>  DNA
<213>  Artificial

<220>
<223>  Synthetic primer

<400>  13
gggagcagtt ttgtggcagc a                                                  21


<210>  14
<211>  23
<212>  DNA
<213>  Artificial

<220>
<223>  Synthetic primer

<400>  14
agggtcctgg gtaaatgggt gag                                                23


<210>  15
<211>  21
<212>  DNA
<213>  Artificial

<220>
<223>  Synthetic primer

<400>  15
tgccgcccac tctccccaac c                                                  21


<210>  16
<211>  21
<212>  DNA
<213>  Artificial

<220>
<223>  Synthetic primer

<400>  16
ccgccgccgt catcgtcttc c                                                  21


<210>  17

<211>  18
<212>  DNA
<213>  Artificial

<220>
<223>  Synthetic primer

<400>  17
tgctgcctgt gagtcata                                                    18


<210>  18
<211>  20
<212>  DNA
<213>  Artificial

<220>
<223>  Synthetic primer

<400>  18
acaagaggct gtagaacatg                                                  20


<210>  19
<211>  19
<212>  DNA
<213>  Artificial

<220>
<223>  Synthetic primer

<400>  19
gctctcctgc tttcccagc                                                   19


<210>  20
<211>  26
<212>  DNA
<213>  Artificial

<220>
<223>  Synthetic primer

<400>  20
ctcccatccc tactgccttc tgcagc                                           26


<210>  21
<211>  18
<212>  DNA
<213>  Artificial

<220>
<223>  Synthetic primer

<400>  21
tccctcatcc tcctgcta                                                    18


<210>  22
<211>  19
<212>  DNA
<213>  Artificial

<220>
<223>  Synthetic primer

<400>  22
gcacattctt ctccgtcac                                                   19

<210>  23
<211>  20
<212>  DNA
<213>  Artificial

<220>
<223>  Synthetic primer

<400>  23
tctacacaga ccaccaacgc                                                    20


<210>  24
<211>  20
<212>  DNA
<213>  Artificial

<220>
<223>  Synthetic primer

<400>  24
agaaactcct ccttgacggg                                                    20


<210>  25
<211>  21
<212>  DNA
<213>  Artificial

<220>
<223>  Synthetic primer

<400>  25
aaagtcaatg gctcccacga a                                                  21


<210>  26
<211>  21
<212>  DNA
<213>  Artificial

<220>
<223>  Synthetic primer

<400>  26
cttcagtctg tacttcactg g                                                  21


<210>  27
<211>  20
<212>  DNA
<213>  Artificial

<220>
<223>  Synthetic primer

<400>  27
actcccactc ttccaccttc                                                    20


<210>  28
<211>  19
<212>  DNA
<213>  Artificial

<220>
<223>  Synthetic primer

<400>  28
tcttgctcag tgtccttgc                                                    19


<210>  29
<211>  30
<212>  DNA
<213>  Artificial

<220>
<223>  Synthetic primer

<400>  29
gattttgtag gtgggattaa ttgtgaattt                                        30


<210>  30
<211>  30
<212>  DNA
<213>  Artificial

<220>
<223>  Synthetic primer

<400>  30
accaaaaaaa cccacactca tatcaatata                                        30


<210>  31
<211>  24
<212>  DNA
<213>  Artificial

<220>
<223>  Synthetic primer

<400>  31
   ggttttttag aggatggttg agtg                                               24


<210>  32
<211>  23
<212>  DNA
<213>  Artificial

<220>
<223>  Synthetic primer

<400>  32
tccaacccta ctaacccatc acc                                               23


<210>  33
<211>  24
<212>  DNA
<213>  Artificial

<220>
<223>  Synthetic primer

<400>  33
aaccaaagga tgaagtgcaa gcgg                                              24


<210>  34
<211>  24
<212>  DNA
<213>  Artificial

<220>
<223>  Synthetic primer

<400>  34
tccaagttgg gttggtccaa gtct                                              24